# EUROPÄISCHE PATENTSCHRIFT

(11) **EP 0 827 956 B1**
(45) Veröffentlichungstag und Bekanntmachung des Hinweises auf die Patenterteilung: **15.01.2003**
(21) Anmeldenummer: 97115339.0
(22) Anmeldetag: 04.09.1997
(51) Int. Cl.: C07D 307/33, C07D 307/32

(54) **Verfahren zur Dehydrierung von 1,4-Butandiol zu Gamma-Butyrolacton**
Process for the dehydrogenation of 1,4-butanediol to gamma-butyrolactone
Procédé de déshydrogénation de 1,4-butanediol en gamma-butyrolactone

(30) Priorität: 05.09.1996 DE 19636066
(43) Veröffentlichungstag der Anmeldung: 11.03.1998
(73) Patentinhaber: BASF AKTIENGESELLSCHAFT, 67056 Ludwigshafen (DE)
(72) Erfinder: Mercker, Hans Jochen, Dr., 68239 Mannheim (DE); Pape, Frank-Friedrich, Dr., 67259 Kleinniedesheim (DE); Simon, Joachim, Dr., 68161 Mannheim (DE); Henne, Andreas, Dr., 67433 Neustadt (DE); Hesse, Michael, Dr., 67549 Worms (DE); Köhler, Ulrich, Dr., 68259 Mannheim (DE); Dostalek, Roman, Dr., 67354 Römerberg (DE); Freire Erdbrügger, Cristina, Dr., 67240 Bobenheim-Roxheim (DE); Kratz, Detlef, Dr., 69121 Heidelberg (DE)
(74) Vertreter: Isenbruck, Günter, Dr.

(56) Entgegenhaltungen:
- EP-A- 0 584 408
- DE-C- 699 945
- GB-A- 1 066 979
- BARTOK, M.; MOLNAR, A.: "Studies on the conversion of diols and cyclic ethers" ACTA CHIM. ACAD. SCI. HUNG., Bd. 100, Nr. 1-4, 1979, Seiten 203-210, XP002049326
- "Ullmann's Encyclopedia of Industrial Chemistry - fifth edition. Vol. A4" 1985 , VCH VERLAGSGESELLSCHAFT , WEINHEIM XP002049327 * Seite 496-497 *
- PATENT ABSTRACTS OF JAPAN vol. 15, no. 1 (C-0793), 7.Januar 1991 & JP 02 255668 A (DAICEL CHEM), 16.Oktober 1990,
- CHEMICAL ABSTRACTS, Band 54, Nr. , Columbus, Ohio, US, Seite , Spalte , Zusammenfassung Nr. 14506, : ""

## Beschreibung

Die Erfindung betrifft ein Verfahren zur Dehydrierung von 1,4-Butandiol zu γ-Butyrolacton in der Gasphase.

Eine Reihe von Verfahren zur katalytischen Dehydrierung von 1,4-Butandiol zu γ-Butyrolacton ist bekannt.

In Ullmanns Encyklopädie der technischen Chemie, Urban & Schwarzenberg, München, 3. Auflage 1953, Band 4, Seiten 802 und 803 sind Verfahren zur Herstellung von γ-Butyrolacton aus 1,4-Butandiol beschrieben. Nach dem Flüssigphaseverfahren wird flüssiges, heißes 1,4-Butandiol bei Normaldruck in einen mit Kupferkontakt (Cu auf Silicagel) gefüllten und auf etwa 200°C erhitzten Kontaktofen gepumpt. Bei dem Dampfphaseverfahren werden die Butandioldämpfe in einem Kreisprozeß mit Wasserstoff bei Normaldruck über einen auf etwa 230 bis 250°C erhitzten Kontakt (Cu auf Bimsstein) geleitet. Das anfallende Rohbutyrolacton enthält neben Tetrahydrofuran und dessen Isomeren Wasser und Spuren von Butyraldehyd. Nachteil des Verfahrens ist die relativ schlechte Dehydrierwirkung des verwendeten Katalysators und der große Anteil an anfallenden Nebenprodukten.

In der JP-A-61-246173 ist die Verwendung von Cu/Cr/Mn-Katalysatoren und Cu/Cr/Zn-Katalysatoren zur Dehydrierung von 1,4-Butandiol beschrieben. Der Katalysator wird beispielsweise aus CuCr₂O₄ und ZnCr₂O₄ erhalten.

Aus der JP-A-02-255668 ist die Verwendung von Cu/Zn-Katalysatoren zur Gasphasendehydrierung von 1,4-Butandiol bekannt.

Die Cu/Cr-Katalysatoren sind aufwendig in der Herstellung und wegen der Verwendung von Chromverbindungen aus Umweltgründen schwierig einzusetzen. Cu/Zn-Katalysatoren weisen in der Regel ein ungenügendes Standzeitverhalten auf.

Aufgabe der vorliegenden Erfindung ist daher die Bereitstellung eines Verfahrens zur Dehydrierung von 1,4-Butandiol zu γ-Butyrolacton, das die Nachteile der bekannten Verfahren vermeidet.

Die Aufgabe wird erfindungsgemäß gelöst durch ein Verfahren zur Dehydrierung von 1,4-Butandiol zu γ-Butyrolacton in der Gasphase in Gegenwart eines Kupfer enthaltenden Katalysators, wobei der Katalysator 4 bis 40 Gew.-% Kupfer und 0 bis 5 Gew.-% Na₂O, bezogen auf das Gesamtgewicht des Katalysators, auf einem Träger aus 80 bis 100 Gew.-% SiO₂ und 0 bis 20 Gew.-% CaO enthält.

Die Verwendung des erfindungsgemäßen Katalysators ist aus technischen, wirtschaftlichen und ökologischen Gründen vorteilhaft. Der erfindungsgemäß eingesetzte Katalysator weist trotz der intrinsisch geringen Dehydrierwirkung des Kupfers eine ausgezeichnete Wirksamkeit auf. Gegenüber bekannten Kupferkontakten auf der Basis von Silicagel, die für die Synthese von γ-Butyrolacton in Flüssigfahrweise verwendet werden, weist der erfindungsgemäße Katalysator eine geringere Verkokungsneigung und eine längere Standzeit auf.

### Katalysator

Der erfindungsgemäße Katalysator zur Dehydrierung von 1,4-Butandiol zu γ-Butyrolacton enthält 4 bis 40 Gew.-%, vorzugsweise 4 bis 32 Gew.-% Kupfer und 0 bis 5 Gew.-%, vorzugsweise 0 bis 2 Gew.-% Na₂O, bezogen auf das Gesamtgewicht des Katalysators, auf einem Träger aus 80 bis 100 Gew.-%, vorzugsweise 90 - 100 Gew.-% SiO₂ und 0 bis 20 Gew.-%, vorzugsweise 0 bis 10 Gew.-% CaO.

Dabei beträgt die Kupferoberfläche auf dem Träger vorzugsweise mehr als 5 m²/g, besonders bevorzugt mehr als 10 m²/g und liegt insbesondere im Bereich von mehr als 10 m²/g bis 50 m²/g.

Die Kupferdispersion beträgt vorzugsweise mehr als 10 %, besonders bevorzugt mehr als 20 % und liegt insbesondere im Bereich von mehr als 20 % bis 50 %.

Vorzugsweise weist der erfindungsgemäße Katalysator eine durchschnittliche Kupferteilchengröße von weniger als 200 nm auf, besonders bevorzugt im Bereich von 5 bis 150 nm, insbesondere im Bereich von 10 bis 100 nm.

Die Kupferoberfläche wird dabei bestimmt, indem eine genau abgewogene Menge an Katalysator nach einstündiger Spülung mit Helium im Vakuum auf 200°C erhitzt und nachfolgend über einen Zeitraum von 16 Stunden mit H₂ reduziert wird. Nach vollständiger Reduktion wird nochmals evakuiert, die Probe auf 35°C erkalten lassen und die Sauerstoffchemisorption durch ein volumetrisches Sorptionsverfahren (Vermessung von mindestens vier definierten Druckpunkten) ermittelt. Unter der Annahme, daß je zwei Kupferatome mit einem Sauerstoffatom reagieren, kann daraus die Kupferoberfläche über den Platzbedarf der Kupferatome ermittelt werden. Relative Vergleiche von Katalysatoren sind damit sehr gut zugänglich.

Aus der Kupferoberfläche und der Konzentration des im Katalysator enthaltenen Kupfers kann die Kupferdispersion errechnet werden. Unter diesem Begriff versteht man das Verhältnis der Anzahl von Kupferatomen an der Oberfläche der Kupferpartikel, d.h. der zugänglichen Kupferatome, zur Gesamtzahl der in den Kupferkristalliten enthaltenen Kupferatome. Diese Gesamtzahl ergibt sich aus dem durch einfache chemische Analytik zugänglichen Gehalt des Katalysators an Kupfer. Zweckmäßigerweise wird dieses Verhältnis normiert.

Die Kupferkristallitgröße kann auch direkt bestimmt werden. Dazu kommt z.B. die Transmissionselektronenmikroskopie in Frage. Die Proben werden in Polymethylmethacrylat fixiert, und es wird ein dünner Schnitt gefertigt (Ultramikrotomie). Vergrößerungen von 500000 erlauben es, auch sehr kleine Partikelgrößen von 1 nm mit dieser präzisen Methode gut zu bestimmen. Zur Ermittlung der Phase der Partikel kann EDXS (Energy Dispersive X-Ray Spectroscopy) oder SAD (Selected Area Diffraction) hinzugezogen werden.

Gemäß einer Ausführungsform der Erfindung enthält der Katalysator 16 bis 32 Gew.-% Kupfer, bezogen auf das Gesamtgewicht des Katalysators, auf einem SiO₂-Träger, mit einer Kupferoberfläche von mehr als 25 m²/g und einer Kupferdispersion von mehr als 20 %. Dabei beträgt die Kupfermenge vorzugsweise etwa 20 Gew.-%.

Gemäß einer Ausführungsform der Erfindung enthält der Katalysator 4 bis 16 Gew.-% Kupfer und 0,5 bis 1 Gew.-% Na₂O, bezogen auf das Gesamtgewicht des Katalysators, auf einem Träger aus 90 bis 95 Gew.-% SiO₂ und 5 bis 10 Gew.-% CaO, mit einer Kupferoberfläche von mehr als 10 m²/g und einer Kupferdispersion von mehr als 20 %. Vorzugsweise beträgt der Gehalt an Kupfer etwa 8 bis 9 Gew.-%, der Anteil an Na₂O 0,5 bis 1 Gew.-% und der Anteil an CaO im Träger etwa 7 Gew.-%.

Der Katalysatorträger ist vorzugsweise ein hochporöser Träger mit einer spezifischen Oberfläche von mehr als 100 m²/g. Als Träger wird SiO₂, gegebenenfalls mit einem Zusatz an CaO verwendet, der vorzugsweise in Form von Perlen, Strängen, Tabletten oder Ringen vorliegt, so daß der Katalysator als Festbett eingesetzt werden kann. Vorzugsweise wird der Träger in Form von Strängen mit einer Dicke von etwa 4 bis 5 mm oder in Form von Perlen mit 3 bis 5 mm Durchmesser eingesetzt. Beispielsweise kann SiO₂ mit einem Anteil an Wollastonit verwendet werden, wobei der Anteil an Wollastonit insbesondere etwa 7 Gew.-% beträgt, bezogen auf den Träger.

### Herstellung des Katalysators

Der erfindungsgemäß verwendete Katalysator kann hergestellt werden durch Tränken des Trägers aus SiO₂ und gegebenenfalls aus CaO mit wäßriger ammoniakalischer Kupfercarbonatlösung und gegebenenfalls wäßriger Natriumnitratlösung.

Die Tränkung erfolgt dabei in überstehender Lösung innerhalb von 15 Minuten. Die getränkten Träger werden dann 5 Stunden bei 120°C getrocknet und danach 2 Stunden bei 350°C kalziniert. Die Tränk- und Kalzinierschritte können mehrfach wiederholt werden, um einen gewünschten Kupfergehalt und gegebenenfalls zusätzlichen Na₂O-Gehalt zu erreichen. Der Kupfergehalt beziehungsweise der Na₂O-Gehalt der Tränklösungen wird dabei so eingestellt, daß nach einmaliger oder mehrmaliger Tränkung der gewünschte Kupfergehalt auf dem Katalysator erhalten wird. Die Tränkung mit Kupfercarbonatlösung und Natriumnitratlösung kann gleichzeitig oder aufeinanderfolgend durchgeführt werden. Der Katalysator enthält nach dem Kalzinieren 5 bis 50 Gew. -% CuO, entsprechend 4 bis 40 Gew.-% Cu, vorzugsweise 5 bis 40 Gew. -% CuO, entsprechend 4 bis 32 Gew. -% Cu, bezogen auf den gesamten Katalysator. Vor der Umsetzung wird der Katalysator im Wasserstoffstrom reduziert, so daß im einsatzfertigen Katalysator vor der Umsetzung elementares Kupfer vorliegt.

### Dehydrierung

Die Dehydrierung von 1,4-Butandiol zu γ-Butyrolacton erfolgt in der Gasphase bei einer Temperatur von 150 bis 300°C, vorzugsweise 180 bis 260°C, besonders bevorzugt 190 bis 230°C. Die Dehydrierung wird bei einem absoluten Druck von 1 bis 40 bar, vorzugsweise 1 bis 10 bar, besonders bevorzugt 1 bis 2 bar, insbesondere drucklos (etwa 1 bar) durchgeführt.

Vorzugsweise wird dabei der Katalysator in Form von Perlen, Strängen, Tabletten oder Ringen als Festbett eingesetzt.

Das erfindungsgemäße Verfahren kann batchweise oder kontinuierlich durchgeführt werden, vorzugsweise kontinuierlich. Bei der kontinuierlichen Verfahrensführung beträgt die Katalysatorbelastung vorzugsweise 0,2 bis 20 kg, besonders bevorzugt 0,5 bis 10 kg, insbesondere 0,5 bis 7,5 kg 1,4-Butandiol pro kg Kupfer pro Stunde.

Vorzugsweise wird die erfindungsgemäße Dehydrierung in Gegenwart von freiem Wasserstoff durchgeführt. Der freie Wasserstoff kann dabei zugesetzt werden, oder es kann der im Laufe der Dehydrierung entstehende freie Wasserstoff eingesetzt werden. Zu Beginn der Dehydrierung wird in der Regel zunächst ein freien Wasserstoff enthaltendes Gas zugeführt, um den Katalysator im Wasserstoffstrom zu reduzieren. Nach Anlaufen der Dehydrierung wird dann der entstehende Wasserstoff teiweise in die Dehydrierung zurückgeführt.

In einer bevorzugten Ausführungsform des erfindungsgemäßen Verfahrens wird 1,4-Butandiol in einem Verdampfer in die Gasphase überführt und mit einem freien Wasserstoff enthaltenden Trägergas durch einen Reaktor geführt, der den Katalysator enthält; nach dem Reaktor wird γ-Butyrolacton auskondensiert und ausgetragen, ein Teil des Trägergases wird ausgetragen, und das verbleibende Trägergas wird als Kreisgas zurückgeführt.

Als Verdampfer für 1,4-Butandiol kann jeder geeignete Verdampfer verwendet werden. Aus dem ausgetragenen Trägergas kann Wasserstoff gewonnen und für weitere chemische Umsetzungen eingesetzt werden. Das anfallende γ-Butyrolacton, das sehr geringe Mengen an 1,4-Butandiol enthält, kann nach der Umsetzung durch übliche Verfahren, beispielsweise durch Destillation oder Extration isoliert und gereinigt werden. Gegebenenfalls anfallende nicht umgesetzte Ausgangsstoffe, insbesondere 1,4-Butandiol können dabei in die Umsetzung zurückgeführt werden.

Nachstehend wird die Erfindung anhand von Beispielen erläutert.

### Versuchsapparatur:

Der nach dem vorstehend beschriebenen Verfahren hergestellte Katalysator wird in einen zylindrischen, elektrisch beheizten Glasreaktor mit einem Volumen von 300 ml eingefüllt, dem 2 mit Raschigringen gefüllte und auf 250°C beziehungsweise 270°C geheizte Verdampfer vorgeschaltet sind.

1,4-Butandiol wird über eine Kolbenpumpe auf den ersten Verdampfer gefahren. Hinter dem Reaktor befinden sich 3 Dimrothkühler, in denen das Reaktionsprodukt, γ-Butyrolacton, auskondensiert wird. 50 Nl/h Reaktionsgas werden über eine Kreisgaspumpe zurückgeführt, wobei überschüssiges Reaktionsgas ausgetragen wird. Zu Beginn der Umsetzung wird der Katalysator im Glasreaktor in einem Gasstrom aus Wasserstoff und gegebenenfalls Stickstoff, der als Frischgasstrom zugeführt wird, reduziert.

### Herstellung von γ-Butyrolacton:

### Beispiel 1:

Bei einer Reaktionstemperatur von 210°C werden 42 g/h 1,4-Butandiol über 250 g eines Katalysators gefahren, der in Form von Strängen mit 4 bis 5 mm Durchmesser im Reaktor vorliegt. Der Katalysator enthält 12 Gew.-% CuO (9,6 Gew.-% Cu) und 0,8 Gew.-% Na₂O, bezogen auf den gesamten Katalysator, auf 93 Gew.-% SiO₂ mit 7 Gew.-% CaO als Träger. Die Kupferoberfläche beträgt mehr als 10 m²/g, die Kupferdispersion mehr als 20%. Die Katalysatorbelastung beträgt 1,75 kg 1,4-Butandiol pro kg Kupfer pro Stunde. Bei einem Umsatz von mehr als 99,9 % beträgt die Selektivität in bezug auf γ-Butyrolacton 98 %. Umsatz und Selektivität bleiben mindestens eine Woche auf gleichem Niveau. Auf den ausgebauten Katalysator wird kein abgeschiedener Kohlenstoff nachgewiesen.

### Beispiel 2:

Bei einer Reaktionstemperatur von 210°C werden 125 g/h 1,4-Butandiol über 200 g eines Katalysators gefahren, der in Form von Strängen mit 4-5 mm Durchmesser im Reaktor vorliegt. Der Katalysator enthält 12 Gew. -% CuO (9,6 Gew.-% Cu) und 0,8 Gew. -% Na₂O, bezogen auf das Gesamtgewicht des Katalysators, auf 93 Gew.-% SiO₂ mit 7 Gew.-% CaO als Träger. Die Katalysatorbelastung beträgt 5,3 kg 1,4-Butandiol pro kg Kupfer pro Stunde. Der Umsatz beträgt mehr als 99,7 %, die Selektivität in bezug auf γ-Butyrolacton 99 %. Umsatz und Selektivität bleiben für mindestens eine Woche auf gleichem Niveau. Auf den ausgebauten Katalysator wird kein abgeschiedener Kohlenstoff nachgewiesen.

### Beispiel 3:

Bei einer Reaktionstemperatur von 210°C werden 42 g/h 1,4-Butandiol über 250 g eines Katalysators gefahren, der in Form von Perlen mit 3 bis 5 mm Durchmesser im Reaktor vorliegt. Der Katalysator enthält 25 Gew.-% CuO (20 Gew. -% Cu) auf SiO₂ als Träger. Die Kupferoberfläche beträgt mehr als 25 m²/g, die Kupferdispersion mehr als 20 %. Die Katalysatorbelastung beträgt 0,8 kg 1,4-Butandiol pro kg Kupfer pro Stunde. Der Umsatz beträgt mindestens 99,5 %, die Selektivität in bezug auf γ-Butyrolacton 98 %. Umsatz und Selektivität bleiben für mindestens 2 Wochen auf gleichem Niveau. Auf dem ausgebauten Katalysator wird kein abgeschiedener Kohlenstoff nachgewiesen.

### Beispiel 4:

Bei einer Reaktionstemperatur von 210°C werden 125 g/h 1,4-Butandiol über 250 g eines Katalysators gefahren, der in Form von Perlen mit 3 bis 5 mm Durchmesser im Reaktor vorliegt. Der Katalysator enthält 25 Gew.-% CuO (20 Gew.-% Cu) auf SiO₂ als Träger. Die Katalysatorbelastung beträgt 2,5 kg 1,4-Butandiol pro kg Kupfer pro Stunde. Der Umsatz beträgt mindestens 99,5 %, die Selektivität in bezug auf γ-Butyrolacton 97 bis 98 %. Umsatz und Selektivität bleiben mindestens eine Woche auf gleichem Niveau. Auf dem ausgebauten Katalysator wird kein abgeschiedener Kohlenstoff nachgewiesen.

## Patentansprüche

1. Verfahren zur Dehydrierung von 1,4-Butandiol zu γ-Butyrolacton in der Gasphase in Gegenwart eines Kupfer enthaltenden Katalysators, **dadurch gekennzeichnet, daß** der Katalysator 4 bis 40 Gew.-% Kupfer und 0 bis 5 Gew.-% Na₂O, bezogen auf das Gesamtgewicht des Katalysators, auf einem Träger aus 80 bis 100 Gew.-% SiO₂ und 0 bis 20 Gew.-% CaO enthält.

2. Verfahren nach Anspruch 1, **dadurch gekennzeichnet, daß** die Kupferoberfläche auf dem Träger mehr als 5 m²/g und die Kupferdispersion mehr als 10 % beträgt.

3. Verfahren nach Anspruch 1 oder 2, **dadurch gekennzeichnet, daß** der Katalysator 16 bis 32 Gew.-% Kupfer, bezogen auf das Gesamtgewicht des Katalysators, auf einem SiO₂-Träger enthält, mit einer Kupferoberfläche von mehr als 25 m²/g und einer Kupferdispersion von mehr als 20 %.

4. Verfahren nach Anspruch 1 oder 2, **dadurch gekennzeichnet, daß** der Katalysator 4 bis 16 Gew.-% Kupfer und 0,5 bis 1 Gew.-% Na₂O, bezogen auf das Gesamtgewicht des Katalysators, auf einem Träger aus 90 bis 95 Gew.-% SiO₂ und 5 bis 10 Gew.-% CaO enthält, mit einer Kupferoberfläche von mehr als 10 m²/g und einer Kupferdispersion von mehr als 20 %.

5. Verfahren nach einem der Ansprüche 1 bis 4, **dadurch gekennzeichnet, daß** die Dehydrierung bei einer Temperatur im Bereich von 150 bis 300°C und bei einem Druck von 1 bis 40 bar durchgeführt wird.

6. Verfahren nach einem der Ansprüche 1 bis 5, **dadurch gekennzeichnet, daß** das Verfahren kontinuierlich durchgeführt wird.

7. Verfahren nach Anspruch 6, **dadurch gekennzeichnet, daß** die Katalysatorbelastung 0,2 bis 20 kg 1,4-Butandiol pro kg Kupfer pro Stunde beträgt.

8. Verfahren nach einem der Ansprüche 1 bis 7, **dadurch gekennzeichnet, daß** die Dehydrierung in Gegenwart von freiem Wasserstoff durchgeführt wird.

9. Verfahren nach einem der Ansprüche 6 bis 8, **dadurch gekennzeichnet, daß** 1,4-Butandiol in die Gasphase überführt wird und mit einem freien Wasserstoff enthaltenden Trägergas durch einen Reaktor geführt wird, der den Katalysator enthält, nach dem Reaktor γ-Butyrolacton auskondensiert und ausgetragen wird, ein Teil des Trägergases ausgetragen wird und das verbleibende Trägergas als Kreisgas zugeführt wird.

10. Katalysator zur Dehydrierung von 1,4-Butandiol zu γ-Butyrolacton, enthaltend 4 bis 40 Gew.-% Kupfer und 0 bis 5 Gew.-% Na₂O, bezogen auf das Gesamtgewicht des Katalysators, auf einem Träger aus 80 bis 100 Gew.-% SiO₂ und 0 bis 20 Gew.-% CaO.

## Claims

1. A process for dehydrogenating 1,4-butanediol to γ-butyrolactone in the gas phase in the presence of a copper-containing catalyst, wherein the catalyst contains 4-40% by weight of copper and 0-5% by weight of Na₂O, based on the total weight of the catalyst, on a carrier consisting of 80-100% by weight of SiO₂ and 0-20% by weight of CaO.

2. A process as claimed in claim 1, wherein the surface area of copper on the carrier is more than 5 m²/g and the copper dispersion is more than 10%.

3. A process as claimed in claim 1 or 2, wherein the catalyst contains 16-32% by weight of copper, based on the total weight of the catalyst, on an SiO₂ carrier, with a surface area of copper of more than 25 m²/g and a copper dispersion of more than 20%.

4. A process as claimed in claim 1 or 2, wherein the catalyst contains 4-16% by weight of copper and 0.5-1% by weight of Na₂O, based on the total weight of the catalyst, on a carrier consisting of 90-95% by weight of SiO₂ and 5-10% by weight of CaO, with a surface area of copper of more than 10 m²/g and a copper dispersion of more than 20%.

5. A process as claimed in any of claims 1 to 4, wherein the dehydrogenation is carried out at a temperature in the range from 150 to 300°C and under a pressure of from 1 to 40 bar.

6. A process as claimed in any of claims 1 to 5, which is carried out continuously.

7. A process as claimed in claim 6, wherein the space velocity is 0.2-20 kg of 1,4-butanediol per kg of copper per hour.

8. A process as claimed in any of claims 1 to 7, wherein the dehydrogenation is carried out in the presence of free hydrogen.

9. A process as claimed in any of claims 6 to 8, wherein 1,4-butanediol is converted into the gas phase and passed with a carrier gas containing free hydrogen through a reactor which contains the catalyst, γ-butyrolactone is condensed out and discharged downstream of the reactor, some of the carrier gas is discharged and the remaining carrier gas is fed in as circulating gas.

10. A catalyst for dehydrogenating 1,4-butanediol to γ-butyrolactone, comprising 4-40% by weight of copper and 0-5% by weight of Na₂O, based on the total weight of the catalyst, on a carrier consisting of 80-100% by weight of SiO₂ and 0-20% by weight of CaO.

## Revendications

1. Procédé de déshydrogénation du 1,4-butanediol en (-butyrolactone en phase gazeuse, en présence d'un catalyseur contenant du cuivre, **caractérisé en ce que** le catalyseur contient 4% à 40% en poids de cuivre et 0% à 5% en poids de Na₂O, par rapport au poids total du catalyseur, sur un support constitué de 80% à 100% en poids de SiO₂ et de 0% à 20% en poids de CaO.

2. Procédé selon la revendication 1, caractérisé en ce la surface du cuivre sur le support s'élève à plus de 5 m²/g et la dispersion du cuivre à plus de 10%.

3. Procédé selon la revendication 1 ou 2, **caractérisé en ce que** le catalyseur contient 16% à 32% en poids de cuivre, par rapport au poids total du catalyseur, sur un support de SiO₂, avec une surface de cuivre supérieure à 25 m²/g et une dispersion du cuivre de plus de 20%.

4. Procédé selon la revendication 1 ou 2, **caractérisé en ce que** le catalyseur contient de 4% à 16% en poids de cuivre et de 0,5% à 1% en poids de Na₂O, par rapport au poids total du catalyseur, sur un support constitué de 90% à 95% en poids de SiO₂ et de 5% à 10% en poids de CaO, avec une surface du cuivre supérieure à 10 m²/g et une dispersion du cuivre de plus de 20%.

5. Procédé selon l'une quelconque des revendications 1 à 4, **caractérisé en ce que** l'on réalise la déshydrogénation à une température comprise dans la gamme de 150°C à 300°C et à une pression de 1 à 40 bars.

6. Procédé selon l'une quelconque des revendications 1 à 5, **caractérisé en ce que** l'on réalise le procédé en continu.

7. Procédé selon la revendication 6, **caractérisé en ce que** la charge du catalyseur est de 0,2 à 20 kg de 1,4-butanediol par kg de cuivre par heure.

8. Procédé selon l'une quelconque des revendications 1 à 7, **caractérisé en ce que** l'on réalise la déshydrogénation en présence d'hydrogène libre.

9. Procédé selon l'une quelconque des revendications 1 à 8, **caractérisé en ce que** l'on transforme le 1,4-butanediol en phase gazeuse et qu'on le fait passer avec un gaz porteur contenant de l'hydrogène libre dans un réacteur contenant le catalyseur, qu'à la sortie du réacteur, la (-butyrolactone se condense et est récupérée, qu'une partie du gaz porteur est évacuée et que le gaz porteur restant sert d'alimentation sous forme de gaz recyclé.

10. Catalyseur pour la déshydrogénation du 1,4-butanediol en (-butyrolactone, contenant de 4% à 40% en poids de cuivre, par rapport au poids total du catalyseur, sur un support constitué de 80% à 100% en poids de SiO₂ et de 0% à 20% en poids de CaO.
